# EUROPEAN PATENT SPECIFICATION

(11) **EP 0 464 096 B1**
(45) Date of publication and mention of the grant of the patent: **26.11.1997**
(21) Application number: 90905219.3
(22) Date of filing: 23.03.1990
(51) Int. Cl.: A61K 39/02

(54) **METHOD OF PREPARING VACCINES**
VERFAHREN ZUR HERSTELLUNG VON VAKZINEN
METHODE DE PREPARATION DE VACCINS

(30) Priority: 23.03.1989 GB 8906795
(43) Date of publication of application: 08.01.1992
(73) Proprietor: MEDICAL RESEARCH INTERNATIONAL LIMITED, Warwickshire B94 6LD (GB)
(72) Inventor: AHMAD, Afshaw, Birmingham B14 7NR (GB); BUCHAN, Alexander, Birmingham B17 0BL (GB); SKINNER, Gordon, Robert, Bruce, Lapworth Warwickshire BG4 6LD (GB)
(86) International application number: GB9000439
(87) International publication number: WO9011087

(56) References cited:
- CH-A- 423 098
- FR-A- 2 388 563
- GB-A- 293 401
- US-A- 2 118 117
- US-A- 3 132 995
- ACTA PATHOLOGICA ET MICROBIOLOGICA SCANDINAVICA, vol. 73, 1968; J.A. MELAND, pp. 413-422/
- PATENT ABSTRACTS OF JAPAN, vol. 10, no. 94 (C-338)(2151), 11 April 1986/
- PATENT ABSTRACTS OF JAPAN, vol. 11, no. 222 (C-435)(2669), 18 July 1987/
- BIOLOGICAL ABSTRACTS, vol. 76, 1983, Philadelphia, PA (US); D.F. AMEND et al., no. 87856/

## Description

This invention relates to a method of preparing vaccines and, more especially to a method of preparing vaccines from prokaryotic micro-organisms. These vaccines may be cross-reactive with other micro-organisms, for example yeasts.

The invention is based on the observation that treatment of an agueous suspension prokaryotic micro-organisms with an organic solvent such as chloroform, diethyl ether, methanol, ethanol or propanol facilitates release of antigenic material from the micro-organisms over a period of time after exposure to the solvent.

This invention provides a method for preparing vaccines which includes the step of mixing an aqueous suspension of prokaryotic micro-organisms with a solvent capable of facilatating release of antigenic material from the micro-organisms discarding the solvent and retaining the treated micro-organisms and/or the aqueous phase containing the treated micro-organisms or antigenic material released therefrom.

As examples of micro-organisms that may be used in the process there may be mentioned Staphylococcus epidermidis, Staphylococcus aureus, Streptococcus viridans, Escherichia coli, Yersinia enterocolytica, Pseudomonas maltophilia and Bacillus cereus.

Following the treatment with the solvent, the treated micro-organisms will, over a period of time, release antigenic material into the aqueous phase. The antigenic material, optionally together with the remainder of the treated micro-organisms, can then be recovered. For immiscibie solvents the recovery procedure may simply comprise separation of the aqueous phase from the solvent phase, optionally with further separation of the solid material from the aqueous phase.

The aqueous phase containing antigenic material and optionally still containing residual solid material, may then be subjected to a drying technique preferably freeze drying.

With miscible solvents recovery will normally comprise removing the water/solvent mixture, preferably by freeze drying optionaliy after separation of residual solid material.

The solvent may however have a deleterious effect on the antigens material and preferably therefore the treated micro-organisms are separated from the solvent as soon as sufficient time has elapsed for the solvent to penetrate the cells of the micro-organisms and loosen the cell structure. For solvents that are immiscible with water it is usually sufficient to mix the solvent with the aqueous suspension and to allow the mixture to stand until the two phases have separated and then to remove and retain the aqueous phase with the treated micro-organisms therein. For solvents that are miscible with water the mixture will normally be allowed to stand for several minutes, preferably at least ten minutes, following which the treated micro-organisms are separated and retained.

Since the treated micro-organisms will release antigenic material into any aqueous medium, including body fluids, the treated micro-organisms themselves comprise a vaccine and may be injected directly into body fluids whereupon the antigenic material will start to be released.

The treated micro-organisms may therefore be separated from the solvent immediately after treatment and either used immediately or retained for future use.

Alternatively, the treated micro-organisms are resuspended in an aqueous medium after separation to release the antigenic material which is then stored in either liquid or solid form.

Due to differences in miscibility of the solvents in water, the procedure is slightly different for different solvents.

With chloroform the bacterial suspension settles on top of the chioroform layer when the two are shaken and left to stand whereas with diethyl ether the layers are reversed; when the suspension of bacteria is shaken with methanol, ethanol or propanol it mixes with the solvents and no phasing effect occurs.

When chloroform or diethyl ether are used the aqueous bacterial suspension can be separated and the bacteria collected by centrifugation; with methanol, ethanol and propanol there is no need to separate the bacterial suspension prior to the centrifugation step. The pellet from centrifugation is resuspended in water or other suitable fluid and may be used directly as a vaccine. Alternatively the suspension may be allowed to stand in order to facilitate release of antigenic material from the bacterial cells into the fluid

The antigenic material may be separated from the higher molecular weight material for example by filtration prior to storage or may be stored as such. The filtered or unfiltered liquid may be stored for short periods at low temperature.

For longer term storage the material is preferably freeze dried.

The following examples illustrates the invention

### Example I

### Method using chloroform

A suspension of bacteria in nutrient broth is mixed with a volume of chloroform sufficient to saturate the aqueous phase, shaken and left to stand for sufficient time to allow clear separation of the two layers. During this time, the chloroform settles at the bottom and the suspension of bacteria remains above an interface. The chloroformed bacterial suspension containing inactivated bacterial cells is then top collected and centrifuged, for example, at 3000 rpm for 15 minutes. As a result, a visible pellet of mlaterial is obtained at the foot of the centrifuge tube. The supernatent is discarded.

The pellet of chloroform treated bacteria is resuspended in an appropriate volume of water or other suitable liquid and left to stand for a period of some hours which results in the progressive release of antigenic material. The bacterial suspension is centrifuged at 3-4,000 rpm for up to ten minutes depending on the density of the micro-organisms. This centrifugation step is necessary to remove a substantial portion of the bacteria which prevents a filter becoming blocked with bacterial cells. The period of time during which the chloroform treated bacteria are pre-incubated prior to filtration can be varied, for example up to 48 hours for certain bacteria; alternatively, the bacterial pellet can be resuspended in water at different time intervals and re-incubated for further periods of time and the filtration procedure repeated; with Staphylococcus epidermidis, the antigenic content of the filtrate was optimal within the first 24 hours at 37^{o}C. Another option consists of pooling the filtered antigens which have been obtained by separate incubations during different time periods.

### Example II

### Method using diethyl ether

The process is essentially the same as that using chloroform except that the bacterial suspension settles at the bottom and the diethyl ether on top. The diethyl ether is removed leaving the ether treated bacterial suspension in the container. The suspension of bacteria is centrifuged and the same procedure as with the chloroformed pellet repeated.

### Example III

### Method using methanol, ethanol or propanol

The bacterial suspension was shaken as previously described with methanol, ethanol or propanol. With these solvents there is no phasing and after standing for an appropriate time, most usually 20 minutes, the suspension of bacteria is centrifuged and the pellet treated as described above.

### Example IV

### Preparation of a vaccine against Staphylococcus epidermidis.

The following example uses chloroform and Staphylococcus epidermidis (Strain '2' as designated in Modun 1989).

A pure culture of Staphylococcus epidermidis grown overnight at 37oC in nutrient broth was concentrated by centrifugation procedures to a concentration of approximately 109 colony-forming units (CFU) per ml. in nutrient broth. This suspension was routinely plated out to confirm the purity of the starting material. A 5ml. volume of this bacterial suspension was mixed with 2mls. of chloroform, shaken and left to stand for 20 minutes. The bacterial suspension was top-collected from the chloroform phase and centrifuged at 3,000 rpm for 15 minutes. A visible pellet of material was obtained, the supernatent discarded and the pellet resuspended in 5 mls. of water. A 100µl. sample of this suspension was tested and found to contain no viable organisms.

This suspension was left for six hours at 37°C to allow release of antigens from the treated bacterial cells. The suspension was centrifuged at 3,000 rpm for ten minutes and the supernatent material filtered through a filter with a pore size of 0.45 micrometer. The filtrate was subjected to ultrasonic vibration for four minutes to break down any residual DNA. This vaccine preparation can be stored at -70° C for short term storage or freeze dried for more long term storage.

### Efficacy of vaccine preparations

### Polypeptide profiles

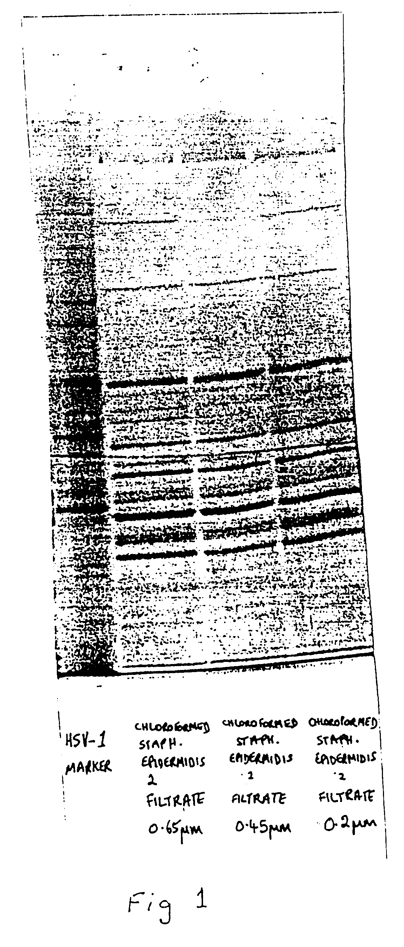

The polypeptides of the vaccine are shown in Fig.1. There were approximately fourteen polypeptides present in the vaccine.

### Antigenicity

Immunoprecipitating antigens were investigated by Outchterlony gel diffusion against hyperimmune antisera raised against crude cell extracts of Staphyloccocus epidermidis. There were at least three immunoprecipitins with one predominant immunoprecipitin (Fig.2).

The total antigenicity of the vaccine was measured by solid phase Elisa assay using hyperimmune antisera (vide supra). The vaccine reacted to an end point titre of 1:10 million This compared favourably to a cell extract antigen prepared by lysostaphin digestion of the bacterial cells which reacted to a titre of 1:10 million. It appeared therefore that there was little loss of antigenicity in the course of vaccine preparation.

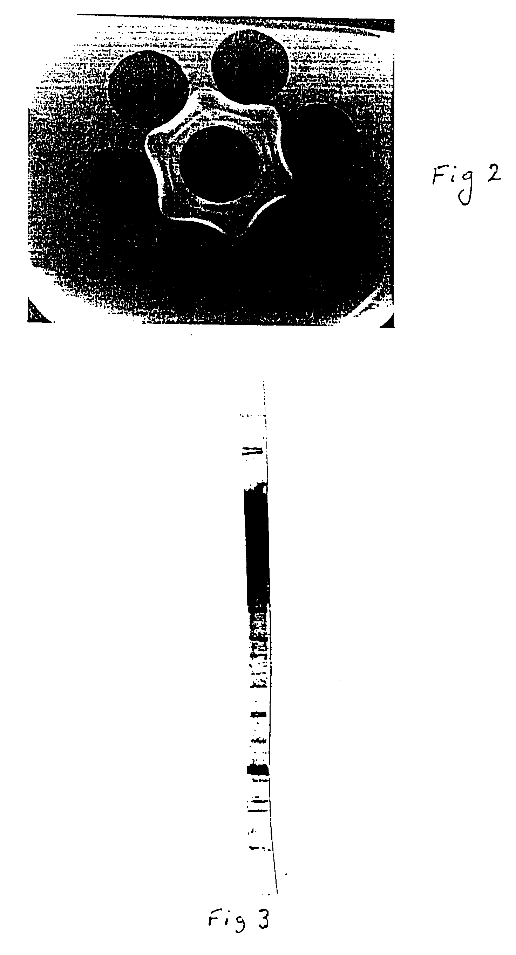

The antigenicity of the polypeptides in the vaccine preparations were examined by immuno blotting against hyperimmune antisera prepared against crude cell extracts prepared by ultrasonic disruption. There were at least 15-20 polypeptides which reacted with the antisera (Fig.3).

Similar antigenicity was observed when a vaccine was prepared by using Staph. epidermidis with diethyl ether, methanol, ethanol or propanol.

### Immunogenicity

A rabbit was injected at three weekly intervals with 10⁷ to 10⁸ bacterial cell equivalents of vaccine. Serum was obtained from the rabbit and tested against extracts of Staph. epidermidis prepared by ultrasonic disruption.

Immunoprecipitating antibodies were sought by Ouchterlony gel diffusion. There were three immunoprecipitin lines with antisera against the vaccine preparation.

Antibody reactivity was investigated by Elisa technique against crude bacterial cell extract prepared by ultrasonic disruption of bacterial cells. Sera obtained following the fourth immunisation of the rabbit reacted to a titre of 1:1,000.

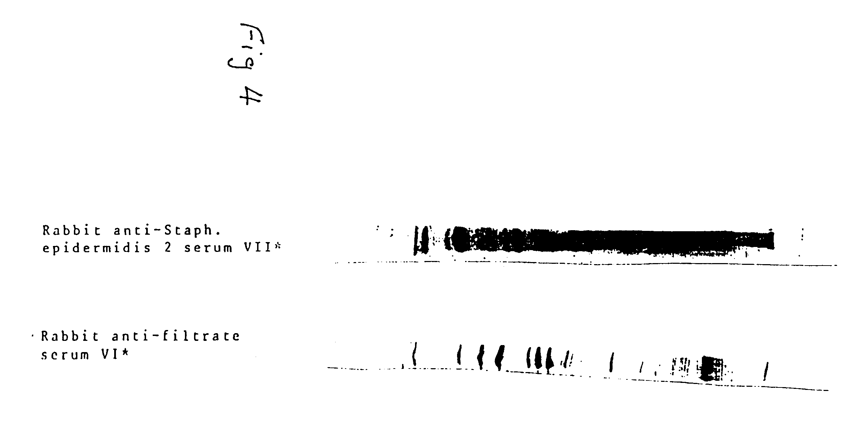

Antibody reactivity was also investigated by immuno-blotting against Lysostaphin-digested bacterial extracts. There were at least twenty polypeptide bands which showed reactivity with molecular weight ranging from as nigh as 153000 to 28000 (Fig.4).

### Bacterial DNA

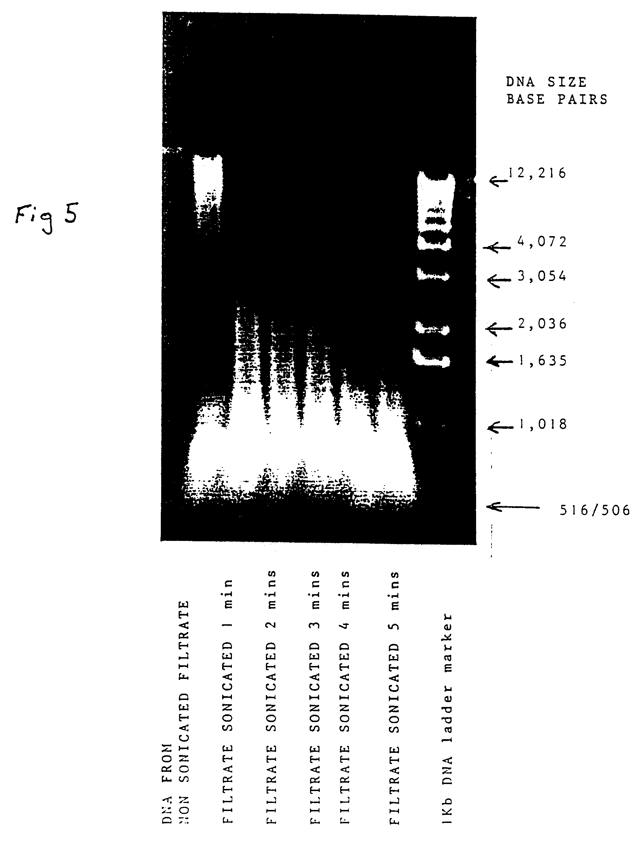

Residual DNA in the vaccine prepration before and after ultrasonication for various periods was analysed by gel electrophoresis on 0.8% agarose and bands of DNA identified by ethidium bromide. There were no discreet DNA bands in the vaccine preparation. There was evidence however, of degraded bacterial DNA and RNA of a size less than 3000 base pairs in the preparation (Fig.5).

### Cross reactivity

Since anti-sera against bacterial extracts of Staph. epidermidis and Staph. aureus cross-react in Ouchterlony gel diffusion with certain bacteria, for example E.coli and Yersinea enterocolytica, cross reactivity was investigated using the antiserum to the vaccine.

Table 1 shows the results of the cross-reactivity tests for hyperimmune sera from whole chloroform treated staph. epidermidis 2 and for filtered chloroform treated Staph. epidermidis 2.

**Table 1**

| Number of immunoprecipitins obtained with hyperimmune serum prepared against whole chloroform treated Staph. epidermidis 2 and various organisms. | |
|---|---|
| Organism | Number of immunoprecipitins |
| S. epidermidis | 3 |
| S. aureus | 1 |
| Streptococcus faecalis | 1 |
| Pseudomonas maltophilia | 1 |
| Psudomonas aeruginosa | 2 |
| Neisseria menignitidis | 1 |
| Proteus mirabilis | 1 |
| Escherichia coli | 1 |
| Clostridium perfringens | 1 |

| Number of immunoprecipitins obtained with hyperimmune serum prepared against filtered chloroform treated S. epidermidis 2 and various organisms. | |
|---|---|
| Organism | Number of immunoprecipitins |
| S. epidermidis | 2 |
| S. aureus | 1 |
| Streptococcus pneumoniae | 1 |
| Psudomonas maetophilia | 1 |
| Pseudomonas aeruginosa | 1 |
| Klebsiella edwardii | 1 |
| Clostridium perfringens | 1 |

Table 2 shows the results of end point dilutions tests with various micro organisms and hyper immune serum against both the whole and the filtered chloroform treated Staph. epidermidis 2.

**Table 2**

| End point dilutions from enzyme linked immunosorbent assays using various organisms and hyperimmune serum against whole chloroform treated S. epidermidis. | |
|---|---|
| Organisms | End point dilutions |
| S. epidermidis | 1/10,000 |
| Streptococcus pneumoniae | 1/2500 |
| Streptocuccus faecalis | 1/1600 |
| Streptococcus pyogenes | 1/1000 |
| Pseudomonas maltophilia | 1/2000 |
| Pseudomonas aeruginosa | 1/400 |
| Escherichia coli | 1/100 |
| Shigella flexnerii | 1/700 |
| Branhamella catarrhalis | 1/1000 |
| Candida albicans | 1/1000 |

| End point dilutions from enzyme linked immunosorbent assays using various organisms and hyperimmune serum against the filtrate of chloroform treated S. epidermidis 2. | |
|---|---|
| Organism | End point dilutions |
| S. epidermidis | 1/1400 |
| Streptococcus pneumoniae | 1/2200 |
| Streptococcus faecalis | 1/300 |
| Streptococcus pyogenes | 1/400 |
| Pseudomonas maltophilia | 1/400 |
| Pseudomonas aeruginosa | 1/60 |
| Escherichia coli | 1/100 |
| Shigella flexnerii | 1/1000 |
| Branharella catarrhalis | 1/200 |
| Candida albicans | 1/300 |

### Protective efficacy

Protective efficacy was examined in CBA mice following four immunisations of whole and filtered chloroform-treated Staphyloccocci epidermidis 2; vaccine was adminstered intraperitoneally at weekly intervals with doses of vaccine derived from approximately 5 x 10⁷ bacterial cells. The mice were challenged with approximately 5 x 10⁸ bacteria by intraperitoneal injection. The frequency of morbidity which consisted of hunching, fur standing on end, partial eye closure and immobility lasting for approximately 24 hours and mortality was recorded in vaccinated and control mice (Table 1). There was a significant reduction in morbidity in vaccinated mice; the rate of mortality was very low with this micro-organism in both control and vaccinated groups of mice.

**Table 3**

| Protective efficacy of whole chloroform treated (WCB) and chloroform treated filtrate (CBF) of S. epidermidis 2 in CBA mice given four weekly injections of the antigen preparation and challenged with live S. epidermidis 2. | | | | |
|---|---|---|---|---|
| Antigen preparation administered x4 at weekly intervals (0.5ml injected IP) | vaccinated | | control | |
| | Morbidity | Mortality | Morbidity | Mortality |
| whole chloroform treated S. epidermidis 2 (WCB) | 4/₁₀ | 0/₁₀ | 20/₂₀ | 15/₂₀ |
| chloroform treated S. epidermidis 2 filtrate (CBF) | 0/₁₀ | 0/₁₀ | 10/₁₀ | 0/₁₀ |
| Total | 4/₂0 | 0/₂₀ | 30/₃₀ | 15/₃₀ |

### Example V

A vaccine was prepared an in Example IV from Staphylococcus aureus and its protective efficacy was tested in similar manner in CBA mice challenged with Staph aureus. The results are shown in Table 4.

**Table 4**

| Protective efficacy in CBA mice; challenged by S.aureus | |
|---|---|
| | Mortality |
| Vaccinated | 5/10 |
| Control | 10/10 |

### Example VI

Ultrasonicated whole chloroform treated Staph. epidermidis 2 was administered to CBA mice as in Exmaple IV but at a dosage rate of 5 x 10⁸cfu/dose. The mice were then challenged with live Pseudomonas aeruginosa. The results are shown in Table 5.

**Table 5**

| Cross protection in CBA mice vaccinated with four weekly doses of ultrasonciated whole chloroform treated S. epidermidis 2 containing the equivalent of 5 x 10⁸ cfu/dose and challenged with live pseudomonas aeruginosa. | | |
|---|---|---|
| Vaccination | Challenge dose of Psudo. aeruginosa | Mortality |
| Ultrasonicated whole chloroform treated S. epidermidis 2 | 1 x 10⁸ to 7 x 10⁷ cfu | 3/₉ |
| Water (control) | | 16/₂₀ |

### Example VII

The vaccine of Example V was tested in similar manner against mice challenged with S. epidermidis. The results are shown in Table 6.

**Table 6**

| Cross protection using whole chloroform treated S. aureus Bate vaccine and challenge with live S. epidermidis 2. | | |
|---|---|---|
| Vaccine | Challenge | Mortality |
| whole chloroform treated S.aureus Bate | S. epidermidis 2 7 x 10⁸ cfu | 0/₅ |
| Control | " | 5/₁₀ |

## Claims

1. A method for preparing vaccines which comprises treating an aqueous suspension of prokaryotic microorganisms with a volume of organic solvent which facilitates release of antigenic material from the microorganisms over a period of time not exceeding 20 minutes after exposure to the solvent, discarding the solvent and retaining the treated microorganisms and/or the aqueous phase containing the microorganisms or antigenic material released therefrom.

2. A method according to claim 1 wherein the organic solvent is chloroform, diethyl ether, methanol, ethanol or propanol.

3. A method according to claim 2 wherein the organic solvent is chloroform or diethyl ether and after treatment of the micro-organisms the aqueous phase is separated from the solvent.

4. A method according to claim 3 wherein the bacterial suspension is mixed with chloroform or diethyl ether and left to stand to allow clear separation of the aqueous layer from the solvent layer for preferably 10 minutes.

5. A method according to claim 3, or claim 4, whenever the solid material is separated from the aqueous phase and resuspended in an aqueous medium to release antigenic material into the aqueous medium.

6. A method according to claim 2, wherein the solvent is methanol, ethanol or propanol and the treated micro-organisms are separated from the liquid phase and resuspended in a aqueous medium to release antigenic material in the aqueous phase.

## Patentansprüche

1. Eine Methode zur Zubereitung von Impfstoffen, die folgendes Verfahren umfasst: die Behandlung einer wässerigen Suspension prokaryontischer Mikroorganismen mit einer gleich großen oder größeren Menge eines organischen Lösungsmittels, die erforderlich ist zur Sättigung der wässerigen Phase, welche die Loslösung von Antigenen über einen gewissen Zeitraum nach dem Ausgesetztsein mit der Lösung erleichtert, indem die Lösung beseitigt wird bei Zurückbehaltung der behandelten Mikroorganismen und/oder der wässerigen Phase, die die Mikroorganismen enthält oder die daraus losgelösten Antigene.

2. Eine Methode gemäß Punkt 1, bei der das organische Lösungsmittel Chloroform Diäthyläther, Methanol, Äthanol oder Propanon ist.

3. Eine Methode gemaß Punkt 2, bei der das organische Lösungsmittel Chloroform oder Diäthyläther ist und bei der die wässerige Phase nach Behandlung der Mikroorganismen vom Lösungsmittel getrennt wird.

4. Eine Methode gemäß Punkt 3, bei der die bakterielle Suspension mit Chloroform oder Diäthyläther vermischt und ruhengelassen wird, so daß die klare Trennung der wässerigen Schicht von der Lösungsmittelschicht ermöglicht wird. Dies dauert typischerweise 10 Minuten.

5. Eine Methode gemaß Punkt 3 oder Punkt 4, bei der, wenn das feste Material von der wässerigen Phase getrennt und neu in einem wässerigen Medium suspendiert wird, Antigene in das wässerige Medium freigesetzt werden.

6. Eine Methode gemaß Punkt 2, bei der das Lösungsmittel wiederum Methanol, Äthanol oder Propanon ist und die behandelten Mikroorganismen von der flüssigen Phase getrennt und neu in einem wässerigen Medium suspendiert werden, um Antigene in der wässerigen Phase freizusetzen.

## Revendications

1. Une méthode de préparation des vaccins dans laquelle une suspension aqueuse de micro-organismes procaryotiques est traitée avec un volume de solvant organique égal ou supérieur à celui nécessaire à la saturation de la phase aqueuse, et qui facilite la libération de substance antigénique à partir de micro-organismes ayant été exposés pendant un certain temps au solvant. Le solvant est ensuite rejeté et on garde les micro-organismes traités et/ou la phase aqueuse contenant les micro-organismes ou la substance d'antigènes ainsi libérée.

2. Une méthode qui en 1 exige que le solvant organique soit du chloroforme, de l'éther diéthyl, du méthanol, de l'éthanol ou du propanol.

3. Une méthode qui en 2 exige que le solvant organique soit du chloroforme ou de I'éther diéthyl et qu'après le traitement des micro-organismes, la phase aqueuse soit séparée du solvant.

4. Une méthode qui en 3 exige que la suspension bactérienne soit mélangée avec du chloroforme ou de l'éther diéthyl et laissée reposée afin de permettre une séparation nette entre la couche aqueuse et la couche de solvant, ce qui demande généralement 10 minutes.

5. Une méthode qui en 3 et 4 exige que la substance solidifiée soit séparée de la phase aqueuse et soit remise en suspension dans un milieu aqueux afin de dégager une substance d'antigènes dans le milieu aqueux.

6. Une méthode qui en 2 exige que ledit solvant soit du méthanol, de l'éthanol ou du propanol et que les micro-organismes traités soient séparés de la phase liquide et remis en suspension dans un milieu aqueux afin de libérer une substance d'antigènes dans la phase aqueuse.
